# EUROPEAN PATENT SPECIFICATION

(11) **EP 0 784 984 B1**
(45) Date of publication and mention of the grant of the patent: **02.07.2003**
(21) Application number: 97100208.4
(22) Date of filing: 09.01.1997
(51) Int. Cl.: A61K 47/48

(54) **Transfection competent molecules**
Transfektionskompetente Moleküle
Molécules compétentes à la transfection

(30) Priority: 17.01.1996 EP 96100603
(43) Date of publication of application: 23.07.1997
(73) Proprietor: F. HOFFMANN-LA ROCHE AG, 4070 Basel (CH)
(72) Inventor: Legendre, Jean-Yves, 75013 Paris (FR); Supersaxo, Andreas, 4052 Basle (CH); Trzeciak, Arnold, 79650 Schopfheim (DE)
(74) Representative: Vergani, Diego, Dr.

(56) References cited:
- EP-A- 0 324 455
- WO-A-93/01828
- WO-A-94/01138
- WO-A-94/09827
- LEGENDRE J.Y. ET AL.: "Short-Chain Phospholipids Enhance Amphipatic Peptide-Mediated Gene Transfer" BIOCHEMICAL AND BIOPHYSICAL JOURNAL, vol. 217, no. 1, 1995, pages 179-185, XP002088443
- AUVIN-GUETTE C. ET AL.: "Trichogin A IV, an 11- Residue Lipopeptaibol from Trichoderma Longibrachiatum" J. AM. CHEM SOC., vol. 114, 1992, pages 2170-2174, XP002088444
- LEGENDRE J.Y.: "Dioleoylmellitin as a Novel Serum- Insensitive Reagent for Efficient Transfection of Mammalian Cells" BIOCONJUGATE CHEMISTRY, vol. 8, 1997, pages 57-63, XP002088445
- DEMPSEY C. E.: "The Actions of Mellitin on Membranes" BIOCHIMICA AND BIOPHYSICA ACTA, vol. 1031, 1990, pages 143-161, XP002088446
- LEGENDRE J-Y.; SZOKA F.C. JR.: 'Cyclic amphipathic peptide-DNA complexes mediate high-efficiency transfection of adherent mammalian cells' PROC. NATL. ACAD. SCI USA vol. 90, 1993, pages 893 - 897

## Description

Gene transfer technology has become a field of considerable interest. Introduction of an exogeneous gene into a cell (i.e. transfection) bears many important scientific and medical applications, going from gene regulation and the production of recombinant proteins to gene therapy.

Viruses have evolved to bypass the different cellular barriers to gene transfer and have indeed become vectors of choice for transfection. Many viruses, including retrovirus, adenovirus or herpes virus, are now engineered to carry therapeutic genes and used in human clinical trials for gene therapy. However, there remains a risk of infectious and immunologic reaction and the large scale production of viruses is difficult and time consuming.

For these various reasons non viral systems have been developed to carry DNA into cells, e.g., the transfection technique based on a cationic lipid, the dioleoyloxypropyl trimethylammonium (Felgner et al., Proc. Natl., Acad. Sci. USA, 84, 7413-7417, 1987) commercialized as Lipofectin™. Since the discovery of this transfection technique, many more cationic lipids have been synthesized and some are commercially available as transfecting reagent for laboratory use: DOGS (Transfectam™), DOSPA (Lipofectamine™), DOTAP (DOTAP™).

Nevertheless, despite an important progress in the formulation of non-viral gene delivery systems, there remains a need for more efficient techniques, since the transfection efficiency of synthetic systems is usually below that of viral vectors. Furthermore, still many problems arise in vivo and the poor stability of the non-viral systems in biological fluids does not allow high and reproducible levels of transfection in vivo.

Transfection of cells with oligonucleotides such as DNA can be used, for example to express in a host cell or organism, a protein which is not normally expressed by that cell or organism. For example, a self replicating DNA molecule called a plasmid may be introduced into a cell not normally containing that plasmid in order to express a marker gene product in that cell, or to express a protein of interest such as a recombinant protein which is later harvested from such cells. (See Sambrook, et al., Molecular Cloning: A Laboratory Manual, 2nd ed. (Cold Spring Harbor, 1989), ch. 1.) The transfection of oligonucleotides into cells can also be used therapeutically. For example, antisense oligonucleotides, once in the cell or cell nucleus, bind to target single-stranded nucleic acid molecules by Watson-Crick base pairing or to double stranded nucleic acids by Hoogsteen base pairing, and in doing so disrupt the function of the target by one of several mechanisms: by preventing the binding of factors required for normal transcription, splicing, or translation; by triggering the enzymatic degradation of mRNA by RNAse H, or by destroying the target via reactive groups attached directly to the antisense oligonucleotide. (See Zamecnic et al., Proc. Natl. Acad. Sci.-USA (1978) 75: 280-284). Gene therapy or DNA based vaccination are other therapeutic applications.

Proteins and other anionic macromolecules are transferred into cells for therapeutic and screening purposes. For example, immunization is enhanced by introducing an immunogenic protein into a cell, so that it is more efficiently processed and presented on the surface of the cells, thereby enhancing the immunogenic response. Negatively charged anionic macromolecules which act inside a cell are transported past the cell membrane into the cytoplasm where they exert their effect. Factors which enhance or hinder transcription of DNA can be used in a screening test to verify the transcription of a gene of interest. These transcription assays are very well known for use in screening compounds to determine their effect against a particular macromolecule, for example a cell receptor.

Legendre J.Y. et al., "Short-Chain Phospholipids Enhance Amphipatic Peptide-Mediated Gene Transfer" Biochemical and Biophysical J., vol. 217, no. 1, 1995, pages 179-185, XP002088443 discloses the use of short-chain phospholipids for enhancing amphipathic peptide-mediated gene transfer.

Dempsey Ch. et al., "The Actions of Mellitin on Membranes", Biochimica and Biophysica Acta, vol. 1031, 1990, pages 143-161, XP002088446 is directed to studies on the action of melittin on membranes. Melittin is a predominently nychophebic membrane-binding peptide.

In accordance with the present invention it has been found that conjugating a lipid to a basic, membrane disturbing peptide results in novel compounds that bind polynucleotides and anionic macromolecules and can be used for transfection of cells. Thus, in one aspect, the invention is concerned with novel compounds which are conjugates of lipids and basic, membrane disturbing peptides and which are transfection competent molecules.

The term "conjugates" means compounds consisting of a lipid chemically bound to the peptide, e.g., via a disulfide bond formed between a sulfur atom present in or attached to the lipid and a sulfur atom present in or attached to the peptide; or an amide bond formed between the carboxyl group present in or attached to the lipid and an amino group of the peptide.

The term "lipid" as used herein comprises straight-chain, branched-chain, saturated or unsaturated aliphatic carboxylic acids and phospholipids. Examples of aliphatic carboxylic acids are lauric acid, palmitic acid, stearic acid, oleic acid and (CH₃(CH₂)ₙ)₂CH COOH, where n is an integer from 3 to 19. Examples of phospholipids are phosphatidylethanolamines such as dioleoylphosphatidylethanolamine.

The term "basic peptides" denotes peptides containing at least one basic amino acid. Examples of such basic amino acids are natural and unnatural diamino-monocarboxylic acids, such as alpha, beta-diaminopropionic acid, alpha, gamma-diaminobutyric acid, lysine, arginine, ornithine and p-aminophenylalanine.

The term "membrane disturbing peptides" denotes cell-lytic or antibacterial peptides which perturb the barrier function of membranes (G. Saberwal and R. Nagaraj BBA 1197:109-131, 1994). Examples of basic, cell-lytic peptides are melittin, hemolysin, mastoparan, bombolitin, crabrolin and derivatives thereof. Examples of basic antibacterial peptides are cecropins, magainins, gramicidin S and tyrocidine and derivatives or analogs thereof. The term "analogs" refers to peptides wherein one or more amino acid residues are missing or have been replaced by another amino acid residue without substantially changing the biological activity of the original peptide concerned. The term "derivatives" refers to peptides wherein the terminal carboxyl group is esterified, particularly to form (C₁-C₆) - alkyl esters such as the methyl and ethyl ester; or converted into an amide, (C₁-C₆) - alkyl amide or di- (C₁-C₆) - alkyl amide or hydrazide.

Thus, in one aspect, the novel compounds of the present invention are compounds of the formula

(R-CONH)ₙ-R³ I

wherein R is the hydrocarbyl moiety of a straight-chain or branched-chain, saturated or unsaturated aliphatic carboxylic acid, or a phospholipid moiety having a free valence bond; R³ is a basic membrane disturbing peptide having a free valence bond at one or two carbon atom(s); and n is 1 or 2.

Preferably, n is 1. Particularly preferred compounds are those of the formula 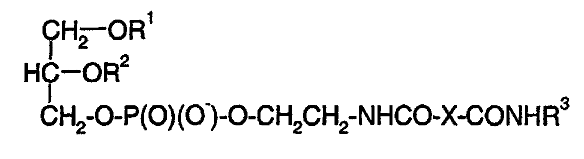 wherein X is C₂₋₁₀ alkylene, R¹ and R² independently are an acyl moiety of a C₁₂₋₂₀ aliphatic carboxylic acid and R³ is as defined above.

The term "C₁₂₋₂₀" denotes a number of carbon atoms of from 12 to 20. The acyl moieties R¹ and R² can be a straight-chain or branched-chain, saturated or unsaturated moiety. Examples of such moieties are lauroyl, palmitoyl, stearoyl and oleoyl. In a preferred aspect, R¹ and R² are oleoyl. X is preferably ethylene, propylene or decamethylene.

In another and preferred aspect, the invention relates to novel compounds of the formula

(R-S-S)ₙ-R³ II

wherein R, R³ and n have the meaning given above.

In the compounds of formula II, n is preferably 1. Most preferred are compounds of the formula 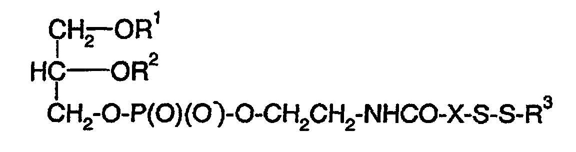 wherein R¹, R², R³ and X are as defined above.

Most preferably R³ is the residue -(CH₂)-CH(NH₂)-CO-Ile-Gly-Ala-Val-Leu-Lys-Val-Leu-Thr-Thr-Gly-Leu-Pro-Ala-Leu-Ile-Ser-Trp-Ile-Lys-Arg-Lys-Arg-Gln-Gln-NH₂.

In another aspect, this invention relates to a process for preparing the novel compounds defined above, i.e., conjugates of lipids and basic, membrane disturbing peptides, and compositions comprising at least one such compound, a polynucleotide or any other anionic macromolecule, and, optionally, a helper lipid and/or a short chain phospholipid, and/or a cationic lipid or another known transfection competent molecule other than a conjugate of this invention (i.e. a compound of formula I or II). In still another aspect, this invention relates to compositions comprising conjugates of lipids and basic, membrane disturbing peptides and a helper lipid and/or a short chain phospholipid, and/or a cationic lipid or another known transfection competent molecule other than a conjugate of this invention, e.g. a compound of formula I or II.. The invention further relates to the use of the novel compounds as a carrier for transfecting a cell with a polynucleotide or any other anionic macromolecule.

The compounds provided by this invention can be prepared by reacting a peptide of the formula R³NH₂ with a lipid of the formula R-COOH, or a peptide of the formula R³SH with a lipid of the formula R-SY wherein Y is a leaving group such as 2-pyridinethio. These reactions can be carried out in a manner known per se.

Thus, the coupling of peptide of the formula R³NH₂ with a lipid of the formula R-COOH can be accomplished by reacting the compounds in a suitable solvent in the presence of a condensation agent such as dicyclohexylcarbodiimide in analogy to methods known for producing peptide bonds.

The reaction of a peptide of the formula R³SH with a compound of formula R-SY can be carried out in an appropriate solvent or solvent mixture which solubilizes both reactants. The compound of formula R-SY can be dissolved in an organic solvent, e.g., in chloroform. The peptide R³SH is suitably dissolved in aqueous buffer solution, such as phosphate buffer, that contains an appropriate amount of an water-miscible organic solvent such as acetonitrile to accomplish the formation of a single phase reaction system.

Compounds of the formula R-COOH and R-SY are known or can be prepared by known methods, e.g. as described in Biochim.Biophys.Acta 862, 435-439 (1986). For example, compounds of the formula 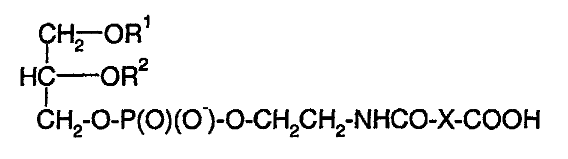 wherein R¹ and R² are oleolyl, and X is ethylene, propylene or decamethylene and the compound of the formula 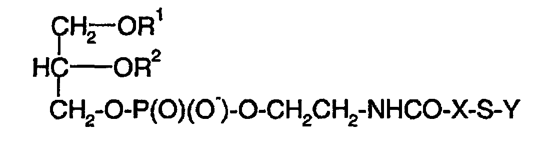 wherein R¹ and R² are oleolyl, X is ethylene and Y is 2-pyridinethio are commercially available as N-Succinyl-PE, N-Glutaryl-PE, N-Dodecanyl-PE and N-PDP-PE from Avanti Polar Lipids, Alabaster, Alabama, USA.

Any anionic macromolecule can be transfected into a cell using a compound of formula I or II. An anionic macromolecule is a macromolecule which contains at least one negative charge per molecule. Examples of anionic macromolecules which can be transfected in accordance with this invention include polynucleotides , such as deoxyribonucleic acids (DNA) and ribonucleic acids (RNA); and proteins, such as ribonucleoproteins and proteins used for immunization, e.g. viral proteins. Examples of DNA for use in the present invention are plasmids and genes, especially those for which gene therapy protocols have been launched such as cystic fibrosis transmembrane regulator (CFTR), adenosine deaminase (ADA), thymidine kinase (tk) and HLA B7; as well as reporter genes such as beta-galactosidase, luciferase, chloramphenicol transferase and alpha-1 antitrypsin. Other examples of DNA are oligodeoxynucleotides and their analogues used as antisense, aptamer or "triple-helix" agents. Examples of RNA are ribozymes or oligoribonucleotide antisense molecules.

The nature of the cell which is to be transfected is not narrowly crucial. The cell can be a procaryotic or eucaryotic cell, a mammalian or a plant cell.

In transfecting a cell using a conjugate of this invention, e.g. a compound of formula I or II, the cell is contacted with the anionic macromolecule in the presence of an appropriate amount of such compound. The appropriate amount of the conjugate, e.g. a compound of formula I or II for a given amount of anionic macromolecule depends on their respective charges. The +/- charge ratio between the conjugate and the molecule to be transfected generally varies between 0.1-10, preferably between 0.5-5. The value of "+/- charge ratio" is calculated by dividing the number of positively charged groups on the amino acids in the group R³ by the number of negative charges of the molecule to be transfected. When the molecule to be transfected is a polynucleotide for example, number of negative charges means the number of negatively charged phosphates in the backbone. The optimal ratio within these ranges depends on the cell to be transfected and is readily ascertained by one of skill in the art to which this invention pertains.

The amount of anionic macromolecules to the number of cells is such that the amount of anionic macromolecule for transfecting 10⁴ cells is from 0.1 ng to 10 µg, preferably from 0.2 µg to 2 µg. When the anionic macromolecule is DNA the preferred amount of DNA for transfecting 10⁴ cells in vitro is from 0.1 µg to 10 µg. When cells are being transfected in vivo, the preferred amount of DNA is from 0.1 µg to 1 g.

In a preferred aspect of the invention the transfection is further carried out in the presence of a helper lipid and/or short chain phospholipid,and/or a cationic lipid or any other known transfection competent molecule other than a conjugate of this invention. Any conventional helper lipid can be used. Helper lipids are phospholipids which are known to increase delivery of macromolecules to cells when used together with known transfection competent molecules. Examples of helper lipids are phospholipids, such as phosphatidylcholine or phosphatidylethanolamines or mixtures thereof. Preferred helper lipids are phosphatidylethanolamines, such as dioleoylphosphatidylethanolamine. Any conventional short chain phospholipid can be used. Short chain phospholipids are phospholipids containing fatty acid residues, which fatty acid residues contain from 6 to 12 carbon atoms in their backbone. Examples of short chain phospholipids are phosphatidylcholines that carry two C₆₋₁₂ fatty acid residues. Preferred short chain phospholipids are dicapryl- and dicapryloyl phosphatidylcholine.

Examples of transfection competent molecules include cationic lipids as described by J.B. Behr in Bioconjugate Chem. 5:382-389 (1994) and X. Gao and L. Huang in Gene Ther. 2:710-722 (1995); polycations as described by A.V. Kabanov and V.A.: Kabanov in Bioconjugate Chem. 6:7-20 (1995); peptides and polymers and other nonviral gene delivery systems as described by F.D. Ledley in Human Gene Therapy 6:1129-1144 (1995).

The helper lipid and/or short chain phospholipid and/or a cationic lipid or another known transfection competent molecule other than a conjugate of this invention is suitably in the form of a liposome, micelles, organic or aqueous dispersions, or organic or aqueous solutions.The optimal molar ratio between the compound of formula I and the helper lipid is 0.1-50, preferably 1-10. The optimal molar ratio between helper lipid and short-chain phospholipid is 2-20. The optimal molar ratio between the compound of formula I or II and additional transfection competent molecules is 0.1-10.

For transfection, an appropriate amount of a conjugate of this invention, e.g., a compound of formula I or II is added to the molecule to be transfected (e.g., plasmid DNA), suitably in an aqueous solution. Optionally, a helper lipid and, if desired, a short chain phospholipid and/or a cationic lipid or another known transfection competent molecule other than a conjugate of this invention is then added, either in form of liposomes, mixed micelles, or as an organic solution or aqueous dispersion. Alternatively, the molecule to be transfected may be added to a composition comprising a compound in accordance with this invention, a helper lipid, and, if desired, a short chain phospholipid and/or a cationic lipid or another known transfection competent molecule other than a conjugate of this invention. The composition may be in solid, liquid, semisolid or aerosol form, suitably in form of liposomes, mixed micelles, or as an organic solution or aqueous dispersion.

For transfecting cells in an animal or human patient the composition can be administered by oral, parenteral (i.v., i.m., s.c., i.d., i.p.) transdermal, pulmonary, nasal, rectal, ocular, ventricular, vascular (catheter) and intratumoral route. Furthermore, the composition can be administered by high velocity impaction administration to the skin surface. The progress of transfection can be measured by appropriate testing protocols which are known to those skilled in the art.

The peptide Cys-Ile-Gly-Ala-Val-Leu-Lys-Val-Leu-Thr-Thr-Gly-Leu-Pro-Ala-Leu-Ile-Ser-Trp-Ile-Lys-Arg-Lys-Arg-Gln-Gln-NH₂ is novel and is also part of the invention. It can be prepared by methods known in the art, e.g., by solid phase peptide synthesis as described below.

The following examples which are not limitative illustrate the invention further.

### Example 1

### a) Preparation of the peptide R³SH

Continuous-flow solid-phase synthesis was performed on a Milligen 9050 synthesizer, starting from Tenta Gel S RAM resin (0.22 mmole/g [Rapp Polymere GmbH, Tübingen, Germany] according to the method described by Atherton and Sheppard, Solid Phase Peptide Synthesis : A Practical Approach (IRL Press Oxford 1989). The base-labile Fmoc group was used for α-amino protection. Side chains were protected with the following protection groups: Arg(Pmc), Cys(Trt), Gln(Trt), Lys(Boc), Ser(But) and Trp(Boc). Fmoc-amino acids (2.5 equiv.) were activated with an equivalent amount of TPTU (Knorr et al. Tetrahedron Lett. 1989, 30,1927-1930) and DIPEA. Fmoc deprotection was achieved with 20% piperidine in DMF. Cys(Trt)-Ile-Gly-Ala-Val-Leu-Lys-Val-Leu-Thr(But)-Thr(But)-Gly-Leu-Pro-Ala-Leu-Ile-Ser(But)-Trp(Boc)-Ile-Lys(Boc)Arg(Pmc)-Lys(Boc)Arg(Pmc)-Gln(Trt)-Gln(Trt)-amide Tenta Gel S-resin (1.1 g) was treated with a mixture (20 ml) of 86% TFE, 10% EDT, 4% H₂O for 3 hours. The reaction mixture was concentrated and poured into diethyl ether and the precipitate was collected by filtration and lyophilized from water. The crude peptide was purified by preparative HPLC. There was obtained homogenous Cys-Ile-Gly-Ala-Val-Leu-Lys-Val-Leu-Thr-Thr-Gly-Leu-Pro-Ala-Leu-Ile-Ser-Trp-Ile-Lys-Arg-Lys-Arg-Gln-Gln-NH₂. 5 TFA.

### b) Preparation of a compound of formula II

The homogenous peptide obtained in paragraph a) above (34.6 mg, 10 µmole) was dissolved in a mixture of 1 ml of 100 mM phosphate buffer, pH 6.5, and 1 ml of acetonitrile. To this solution there was added 10.6 mg of 1,2-dioleoyl-sn-glycero-3-phosphoethanolamine-N-[3-(2-pyridyldithio)propionate] in 2.8 ml of chloroform. The mixture was left to stand at room temperature for 1 hour and the organic solvent was removed by evaporation.The remaining solution was diluted with water to a volume of 2.5 ml and passed through a Pharmacia Biotech PD-10 column. The product was eluted with 3.5 ml of water and lyophilized. There was obtained 28.9 mg of a the compound of formula IIA wherein R¹ and R² are oleoyl and R³ is the residue -(CH₂)-CH(NH₂)-CO-Ile-Gly-Ala-Val-Leu-Lys-Val-Leu-Thr-Thr-Gly-Leu-Pro-Ala-Leu-Ile-Ser-Trp-Ile-Lys-Arg-Lys-Arg-Gln-Gln-NH2. ISP-MS:M=3722.

### Example 2

a) 1.1 mg of the compound obtained in Example 1b) were solubilized in trifluoroethanol and mixed with 1.1 mg of dioleoylphosphatidylethanolamine (DOPE) in chloroform. The mixture was dried in vacuo and the remaining film rehydrated with 1 ml of 30 mM Tris Cl buffer pH 8.5.
b) Plasmid DNA (10 µg) was diluted in 400 µl of 30 mM Tris Cl buffer pH 8.5. Then 20 µl of the product obtain in paragraph a) were added and gently mixed. The mixture was then added to cells.

Table 1 shows the transfection efficiency of the compound obtained in Example 1b formulated according to Example 2 and that of two commercially available transfection vectors, DOTAP and Lipofectamine in various cell lines using the same transfection conditions.

The effect of DNA dose on the transfection efficiency of various cell lines using the compound obtained in Example 1b formulated according to the example 2 is shown in Table 2. The data indicate that high doses of DNA and the compound obtained in Example 1b could be applied on cells without any obvious toxicity.

**Table 1 :**

| Transfection of various cell lines with a mixture of the compound of Example 1b and DOPE or cationic lipids. Cells were transfected with a β-galactosidase encoding plasmid formulated as described earlier in this Example. β-galactosidase activity was measured 48 h after transfection. Results are expressed as µUnits per well, | | | |
|---|---|---|---|
| Cell-line | Luciferase activity | | |
| | Compound of Example 1b | DOTAP ²⁾ | Lipofectamine ²⁾ |
| 293-EBNA | 2343658 | 706718 | 99050 |
| C2E12 | 563827 | 156947 | 17655 |
| CHO K1 | 1825019 | 814133 | 48457 |
| HeLa | 33497 | 5409 | 243 |
| LM | 31685 | 68614 | 35066 |
| LM tk- | 1712739 | 594518 | 45435 |

| | | | |
|---|---|---|---|
| ²⁾ DOTAP and Lipofectamine transfection complexes were prepared according to the manufacturer's instructions. | | | |

**Table 2 :**

| Transfection of various cell lines with a mixture of the compound of Example 1b and DOPE using increasing DNA doses. Cells were transfected with a β-galactosidase encoding plasmid formulated as described earlier in this Example. β-galactosidase activity was measured 48 h after transfection. Results are expressed as µUnits per well, | | | | |
|---|---|---|---|---|
| | β-Galactosidase activty | | | |
| | COS-1 | HepG2 | CV-1 | 3T3 |
| 0.2 µg DNA | 16325 | 2533 | 2700 | 318 |
| 0.5 µg DNA | 20545 | 7944 | 4440 | 451 |
| 1 µg DNA | 16005 | 8988 | 9775 | 1032 |
| 2 µg DNA | 15740 | 13816 | 20434 | 1301 |

### Example 3

a) 1.0 mg of the compound obtained in Example 1b) was dissolved in trifluoroethanol and dried in vacuo. The film was then rehydrated with 1 ml of 10 mM Tris maleate buffer pH 6.
b) 10 µl of a plasmid DNA solution (1 mg/ml) were diluted with 190 µl of pure, sterile water . 100 µl of the product obtained in paragraph a) were added and gently mixed. The mixture was then added to cells.

Table 3 shows the transfection efficiency of the compound of Example 1b formulated as described above and that of two commercially available cationic lipid transfection reagents, DOTAP and DOSPER in various cell lines using the same transfection conditions. The data indicate that the compound of Example 1b mediated 3 to 40 and 25 to 500 times greater luciferase activity than DOSPER and DOTAP, respectively. Moreover, only the compound of Example 1b was able to transfect the suspension cell-line WEHI 231.7, albeit at a low level.

**Table 3 :**

| | | | |
|---|---|---|---|
| Transfection of various cell lines with the compound of Example 1b or cationic lipids. Cells grown in 6 well plates were transfected with a luciferase encoding plasmid (2 µg DNA per well) formulated as described earlier in this Example. Luciferase activity was measured 48 h later. Results are expressed as light units per mg of cell protein. Background was substracted from each measurement. | | | |

| **Cell line** | **Luciferase activity** | | |
|---|---|---|---|
| | **DOTAP*** | **DOSPER*** | **Compound of Example 1b** |
| CHO-K1 | 2.69 10⁶ | 2.50 10⁷ | 5.19 10⁸ |
| CV-1 | 1.14 10⁵ | 1.31 10⁶ | 5.54 10⁷ |
| 293 | 7.31 10⁶ | 5.70 10⁷ | 1.71 10⁸ |
| WEHI 231.7 | 0 | 0 | 1.85 10² |

| | | | |
|---|---|---|---|
| *) DOTAP (Avanti Polar Lipids Inc.) or DOSPER (Boehringer Mannheim) transfection complexes were prepared according to manufacturer's instructions and were used at a cationic lipid / DNA ratio of 5/1 (wt/wt). | | | |

### Example 4

a) 1.0 mg of the compound obtained in Example 1b) was dissolved in trifluoroethanol and dried in vacuo. The film was then rehydrated with 1 ml of 10 mM Tris maleate buffer pH 6.
b) 10 µl of a plasmid DNA solution (1 mg/ml) were diluted with 200 µl of pure, sterile water. 39 µl of the product obtained in paragraph a) and 14 µl of an aqueous polyethylenimine solution (0.45 mg/ml) were added. The mixture was adjusted with water to 300 µl, gently mixed and then added to 293 EBNA cells.
c) 10 µl of a plasmid DNA solution (1 mg/ml9 were diluted with 200 µl of pure, sterile water. 52 µl of the product obtained in paragraph a) were added and adjusted with water to 300 µl. The mixture was gently mixed and then added to 293 EBNA cells.
d) 10 µl of a plasmid DNA solution (1 mg/ml) were diluted with 200 µl of pure, sterile water. 56 µl of an aqueous polyethylenimine solution (0.45 mg/ml) were added and adjusted with water to 300 µl. The mixture was gently mixed and then added to 293 EBNA cells.

Table 4 shows the transfection efficiency of the compound of Example 1b, polyethylenimine and a mixture thereof formulated earlier in this Example. The data indicate that the mixture mediated 250 and 25 times greater luciferase activity than compound of Example 1b and polyethlyenimine, respectively.

**Table 4 :**

| | |
|---|---|
| Transfection of 293 EBNA cells with the compound of Example 1b, polyethylenimine or a mixture thereof. Cells grown in 6 cm dishes were transfected with a luciferase encoding plasmid (1 µg DNA per well) formulated as described earlier in this Example. Luciferase activity was measured 48 h later. Results are expressed as relative light units per dish, | |

| **Transfection agent** | **Luciferase activity** |
|---|---|
| Compound of Example 1b | 3.9 x 10⁵ |
| Polyethylenimine | 3.8 x 10⁴ |
| Compound of Example 1b / Polyethylenimine | 9.8 x 10⁶ |

## Claims

1. Conjugates of lipids and basic, membrane disturbing peptides , which are compounds of the formula
(R-CONH)ₙ-R³ I
or of the formula
(R-S-S)ₙ-R³ II
wherein R is the hydrocarbyl moiety of a straight-chain or branched-chain, saturated or unsaturated aliphatic carboxylic acid, or a phospholipid moiety having a free valence bond; R³ is a basic membrane disturbing peptide having a free valence bond at one or two carbon atom(s); and n is 1 or 2.

2. Conjugates as in claim 1 having the formula 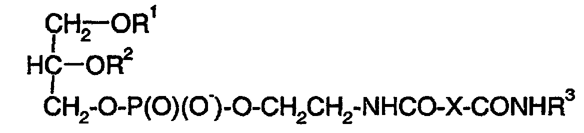 wherein X is C₂₋₁₀ alkylene, R¹ and R² independently are an acyl moiety of a C₁₂₋₂₀ aliphatic carboxylic acid and R³ is as defined in claim 1.

3. Conjugates as in claim 1 having the formula 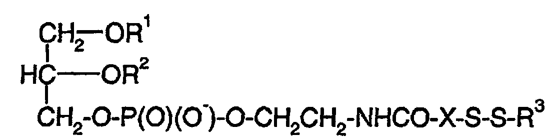 wherein R¹, R², R³ and X are as defined in claims 1 and 2.

4. The conjugate of claim 3, wherein R¹ and R² are oleoyl and R³ is the residue -(CH₂)-CH(NH₂)-CO-Ile-Gly-Ala-Val-Leu-Lys-Val-Leu-Thr-Thr-Gly-Leu-Pro-Ala-Leu-Ile-Ser-Trp-Ile-Lys-Arg-Lys-Arg-Gln-Gln-NH₂.

5. The peptide, Cys-Ile-Gly-Ala-Val-Leu-Lys-Val-Leu-Thr-Thr-Gly-Leu-Pro-Ala-Leu-Ile-Ser-Trp-Ile-Lys-Arg-Lys-Arg-Gln-Gln-NH₂ and derivatives thereof wherein the amino groups are protected.

6. A composition comprising at least one conjugate as defined in any one of claims 1-4, a polynucleotide or any other anionic macromolecule, and, optionally, a helper lipid and/or a short chain phospholipid and/or a cationic lipid or another known transfection competent molecule other than a conjugate of this invention.

7. A composition comprising at least one conjugate as defined in any one of claims 1-4 and, optionally, a helper lipid and/or a short chain phospholipid and/or a cationic lipid or another known transfection competent molecule other than a conjugate of this invention.

8. A composition as in claim 6 or 7, wherein the components are in the form of an aqueous or organic solution, an aqueous or organic dispersion, or a liposome or a micelle.

9. A composition as in claim 6 or 7, wherein the composition is in solid, liquid, semisolid or aerosol form.

10. The use of a conjugate as defined in any one of claims 1-4 and, optionally a helper lipid and/or a short chain phospholipid and/or a cationic lipid or another known transfection competent molecule other than a conjugate of this invention for the manufacture of a gene delivery system suitable as vector for transfecting a cell with a polynucleotide or any other anionic macromolecule.

## Patentansprüche

1. Konjugate von Lipiden und basischen, membranstörenden Peptiden, welche Verbindungen der Formel
(R-CONH)ₙ-R³ I
oder der Formel
(R-S-S)ₙ-R³ II
sind, wobei R die Kohlenwasserstoffeinheit einer geradkettigen oder verzweigten, gesättigten oder ungesättigten aliphatischen Carbonsäure, oder eine Phospholipideinheit ist, welche eine freie Valenzbindung aufweist; R³ ein basisches, membranstörendes Peptid ist, welches eine freie Valenzbindung an einem oder zwei Kohlenstoffatom(en) aufweist; und n 1 oder 2 ist.

2. Konjugate gemäß Anspruch 1 der Formel 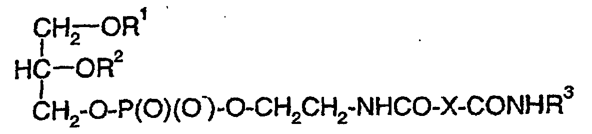 wobei X ein C₂₋₁₀-Alkylenrest ist, R¹ und R² unabhängig voneinander eine Acyleinheit einer aliphatischen C₁₂₋₂₀-Carbonsäure sind und R³ die in Anspruch 1 angegebene Bedeutung hat.

3. Konjugate gemäß Anspruch 1 der Formel 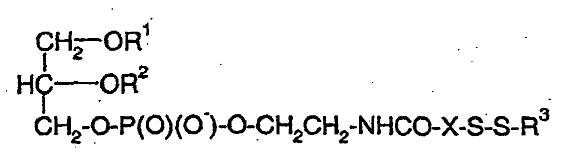 wobei R¹, R², R³ und X die in den Ansprüchen 1 und 2 angegebene Bedeutung haben.

4. Konjugat gemäß Anspruch 3, wobei R¹ und R² eine Oleoylgruppe sind und R³ der Rest -(CH₂)-CH(NH₂)-CO-Ile-Gly-Ala-Val-Leu-Lys-Val-Leu-Thr-Thr-Gly-Leu-Pro-Ala-Leu-Ile-Ser-Trp-Ile-Lys-Arg-Lys-Arg-Gln-Gln-NH₂ ist.

5. Peptid Cys-Ile-Gly-Ala-Val-Leu-Lys-Val-Leu-Thr-Thr-Gly-Leu-Pro-Ala-Leu-Ile-Ser-Trp-Ile-Lys-Arg-Lys-Arg-Gln-Gln-NH₂ und Derivate davon, wobei die Aminogruppe geschützt ist.

6. Zusammensetzung, umfassend mindestens ein Konjugat gemäß einem der Ansprüche 1 bis 4, ein Polynukleotid oder ein anderes anionisches Makromolekül und, gegebenenfalls, ein Hilfslipid und/oder ein kurzkettiges Phospholipid und/oder ein kationisches Lipid oder ein anderes bekanntes transfektionskompetentes Molekül verschieden von einem Konjugat dieser Erfindung.

7. Zusammensetzung, umfassend mindestens ein Konjugat gemäß einem der Ansprüche 1 bis 4 und, gegebenenfalls, ein Hilfslipid und/oder ein kurzkettiges Phospholipid und/oder ein kationisches Lipid oder ein anderes bekanntes transfektionskompetentes Molekül verschieden von einem Konjugat dieser Erfindung.

8. Zusammensetzung gemäß Anspruch 6 oder 7, wobei die Bestandteile in Form einer wässrigen oder organischen Lösung, einer wässrigen oder organischen Dispersion, oder eines Liposoms oder einer Mizelle sind.

9. Zusammensetzung gemäß Anspruch 6 oder 7, wobei die Zusammensetzung in fester, flüssiger, halbfester oder Aerosolform ist.

10. Verwendung eines Konjugats gemäß einem der Ansprüche 1 bis 4 und, gegebenenfalls, eines Hilfslipids und/oder eines kurzkettigen Phospholipids und/oder eines kationischen Lipids oder eines anderen bekannten transfektionskompetenten Moleküls verschieden von einem Konjugat dieser Erfindung zur Herstellung eines Gentransportsystems, welches als Vektor zur Transfektion einer Zelle mit einem Polynukleotid oder einem anderen anionischen Makromolekül geeignet ist.

## Revendications

1. Conjugués de lipides et de peptides perturbant la membrane, basiques, qui sont des composés de formule
**(R-CONH)**_{**n**}**-R**^{**3**} **I**
ou de formule
**(R-S-S)**_{**n**}**-R**^{**3**} **II**
dans laquelle R est le résidu hydrocarbyle d'un acide carboxylique aliphatique saturé ou insaturé, à chaîne linéaire ou ramifiée, ou un résidu phospholipidique ayant une valence libre, R³ est un peptide perturbant la membrane, basique, ayant une valence libre, au niveau d'un ou deux atome(s) de carbone ; et n vaut 1 ou 2.

2. Conjugués selon la revendication 1 répondant à la formule 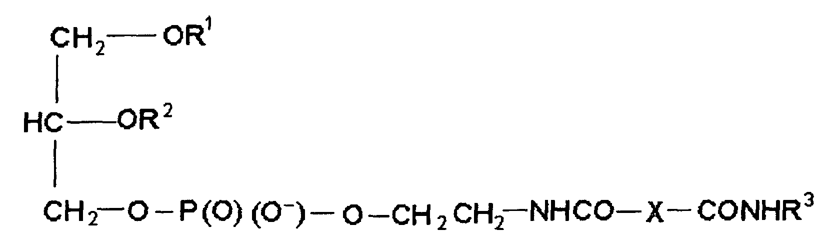 dans laquelle X est un groupe alkylène en C₂ à C₁₀, R¹ et R² sont indépendamment un résidu acyle d'un acide carboxylique aliphatique en C₁₂ à C₂₀ et R³ est tel que défini dans la revendication 1.

3. Conjugués selon la revendication 1, répondant à la formule 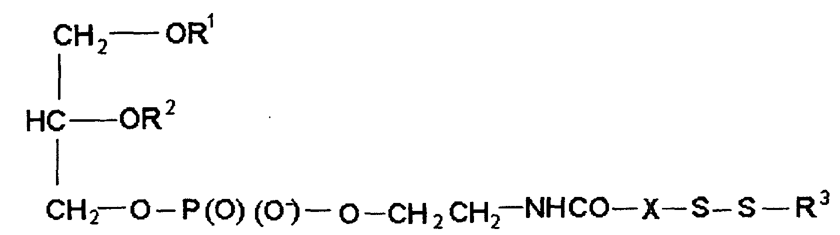 dans laquelle R¹, R², R³ et X sont tels que définis dans les revendications 1 et 2.

4. Conjugué selon la revendication 3, dans lequel R¹ et R² sont des groupes oléoyle et R³ est l'entité -(CH₂)-CH(NH₂)-CO-Ile-Gly-Ala-Val-Leu-Lys-Val-Leu-Thr-Thr-Gly-Leu-Pro-Ala-Leu-Ile-Ser-Trp-Ile-Lys-Arg-Lys-Arg-Gln-Gln-NH₂.

5. Peptide, Cys-Ile-Gly-Ala-Val-Leu-Lys-Val-Leu-Thr-Thr-Gly-Leu-Pro-Ala-Leu-Ile-Ser-Trp-Ile-Lys-Arg-Lys-Arg-Gln-Gln-NH₂ et les dérivés correspondants dans lesquels les groupes amino sont protégés.

6. Composition comprenant au moins un conjugué tel que défini dans l'une quelconque des revendications 1 à 4, un polynucléotide ou toute autre macromolécule anionique, et, éventuellement, un lipide auxiliaire et/ou un phospholipide à chaîne courte et/ou un lipide cationique ou tout autre molécule compétente en transfection connue autre qu'un conjugué de cette invention.

7. Composition comprenant au moins un conjugué tel que défini dans l'une quelconque des revendications 1 à 4, et, éventuellement, un lipide auxiliaire et/ou un phospholipide à chaîne courte et/ou un lipide cationique ou une autre molécule compétente en transfection connue autre qu'un conjugué de cette invention.

8. Composition selon la revendication 6 ou la revendication 7, dans laquelle les composants existent sous forme d'une solution aqueuse ou organique, d'une dispersion aqueuse ou organique, ou d'un liposome ou d'une micelle.

9. Composition selon la revendication 6 ou la revendication 7, dans laquelle la composition est sous forme solide, liquide, semi-solide ou d'un aérosol.

10. Utilisation d'un conjugué selon l'une quelconque des revendications 1 à 4 et, éventuellement d'un lipide auxiliaire et/ou d'un phospholipide à chaîne courte et/ou d'un lipide cationique ou d'une autre molécule compétente en transfection connue autre qu'un conjugué de cette invention pour l'obtention d'un système de délivrance de gènes adapté comme vecteur de transfection d'une cellule par un polynucléotide ou toute autre macromolécule anionique.
